# EUROPEAN PATENT APPLICATION

(11) **EP 4 252 907 A1**
(43) Date of publication of application: **04.10.2023**
(21) Application number: 22165559.0
(22) Date of filing: 30.03.2022
(51) Int. Cl.: B01L 3/00, B01L 9/00, B01L 7/00, G01N 1/00, C12Q 1/6806

(54) **A METHOD FOR ARRANGING MICROSPHERES IN A NON-AQUEOUS LIQUID PHASE**

(71) Applicant: Blink AG, 07747 Jena (DE)
(72) Inventor: ERMANTRAUT, Eugen, 07749 Jena (DE); WOLFF, Alrik, 99425 Weimar (Tiefurt) (DE); LONCAREVIC BARCENA, Ivan, 07743 Jena (DE); BOCKER, Hartmut, 07743 Jena (DE); WAGNER, Cornelius, 07743 Jena (DE); SCHULZ, Torsten, 07749 Jena (DE)
(74) Representative: Engelhard, Markus

(57) **Abstract**

The present invention relates to a method for arranging microspheres in a non-aqueous, water-immiscible liquid phase in an incubation chamber and for excluding such microspheres from contact with or exposure to a gas phase. It also relates to a method for incubating microspheres, as well as to a method for detecting and optically probing microspheres. Moreover, the present invention also relates to a multi-well plate configured to be used in a such method(s). Furthermore, the present invention relates to a device for arranging microspheres in a non-aqueous, water-immiscible liquid phase in an incubation chamber and for excluding such microspheres from contact with or exposure to a gas phase. Furthermore, the present invention also relates to a device for incubating microspheres, as well as to a device for detecting and optically probing microspheres.

## Description

The present invention relates to a method for arranging microspheres in a non-aqueous, water-immiscible liquid phase in an incubation chamber and for excluding such microspheres from contact with or exposure to a gas phase. Moreover, the present invention also relates to a multi-well plate configured to be used in a such method. Furthermore, the present invention relates to a device for arranging microspheres in a non-aqueous, water-immiscible liquid phase in an incubation chamber and for excluding such microspheres from contact with or exposure to a gas phase.

Microspheres, commonly, and herein also sometimes refer to as "nanoreactor beads" are a novel class of molecular biology reagents providing for a wide range of possible applications, such as nucleic acid extraction, digital nucleic acid amplification, e.g. digital polymerase chain reaction and detection. In their most simple configuration, such microspheres or "nanoreactor beads" comprise a hydrogel that provides for a defined volume that acts as a reaction space for a biochemical reaction to take place. In the context of nucleic acid analysis, the hydrogel is used to take up the reaction components (e. g. buffer, amplification reagents, etc.) and the target material to be amplified. Upon loading the hydrogel with the reagent mix, the microspheres are encapsulated in an oil-phase, as a result of which, the respective volume of the individual microsphere functions as an isolated reaction space with no cross-reactivity with other microspheres/nanoreactors. At this stage it is then possible to expose the oil-encapsulated microspheres to suitable amplification reaction conditions, such as specific temperature profiles to which the microspheres are exposed.

Since each individual microsphere provides an isolated volume, it is important to ensure uniform conditions across all microspheres. Typically, this is achieved by incubating the entire vessel/container in which the microspheres reside, until equilibrium conditions, e. g. an equilibrium temperature, is/are reached within such vessel/container. However, this approach is rather slow as reflected by classical digital PCR thermocycling protocols.

Moreover, due to differences between the oil phase and the microspheres and due to immiscibility between ab oil phase and an aqueous phase, the microspheres containing an aqueous phase concentrate and tend to coalesce at elevated temperatures, due to the resulting forces. Furthermore, the microspheres are likely to accumulate at an interface formed between the oil phase and the surrounding atmosphere which may result in drying artefacts.

Once the amplification reaction is complete, the microspheres are examined for the presence of amplified targets, and this occurs by a suitable optical detection device. However, in-situ detection is cumbersome and requires complex techniques, such as a light sheet imaging (Liao et al., PNAS USA, 2020, 117 (41), pp. 25628-25633). Alternatively, for detection upon completion of amplification, the microspheres can be transferred to a reader similar to a flow cytometer (Hindson et al. Anal. Chem., 2011, 83(22): pp. 8604-8610; Kiss et al. Anal. Chem. 2009, 80(23): pp. 8975-8981). Alternatively, to avoid the aforementioned problems, microfluidic techniques have been developed to generate and accommodate droplets in microfluidic devices, providing for an orderly arranged array of droplets, thus aiming at a uniform exposure to identical conditions and at a simplified read out by imaging the droplets arranged in a to-dimensional fashion (Madic et al., Biomol. Detect. Quantif. 2016, 10: pp. 34-46).

Therefore, there remains a need in the art to reduce disposable complexity and to provide for a cost-effective and safe method for accommodating microspheres/nanoreactor beads in an arrangement suitable for amplification and detection. There is also a need in the art to provide for a methodology that avoids the aforementioned disadvantages and problems associated with the prior art. In particular, there is a need to provide for a method for arranging microspheres in a suitable arrangement and to exclude such microspheres from contact with or exposure to a gas phase.

In a first aspect, the present invention relates to a method for arranging microspheres in a non-aqueous, water-immiscible liquid phase in an incubation chamber and for excluding such microspheres from contact with or exposure to a gas phase, said method comprising the steps:
a) Providing, in any order, a suspension comprising magnetic microspheres suspended in a non-aqueous, water-immiscible liquid phase, and an incubation chamber;
   said incubation chamber having a closed bottom, an open top, at least one wall, and a defined volume; said open top being spaced apart from said closed bottom by a defined distance; said chamber being dimensioned to accommodate a defined volume of said suspension therein;
   said magnetic microspheres having a first defined density; said non-aqueous, water-immiscible liquid phase having a second defined density, wherein said first defined density < said second defined density; and wherein, preferably, said suspension has a defined concentration of microspheres;
b) Filling said incubation chamber with said suspension by placing said suspension into said incubation chamber, such that in said incubation chamber, at the open top or at a first position along said defined distance between said open top and said closed bottom, there is formed a first interface between said non-aqueous, water-immiscible liquid phase and a gas phase, such gas phase surrounding said incubation chamber;
c) Removing said magnetic microspheres from said first interface or keeping them removed therefrom by attracting said magnetic microspheres to the closed bottom of said incubation chamber or to said at least one wall, wherein said attracting is achieved by exerting a magnetic force across said closed bottom or across said at least one wall of said incubation chamber;
d) Whilst keeping said magnetic microspheres attracted to the closed bottom or to said at least one wall of said incubation chamber by continuing to exert a magnetic force across said closed bottom or said at least one wall, placing a solid surface at said open top of said incubation chamber or at a position along said defined distance between said open top and said closed bottom of said incubation chamber; wherein such placing occurs such that said solid surface is in contact with said liquid phase and such that at said open top or at said position there is formed a second interface between said non-aqueous, water-immiscible liquid phase and said solid surface, wherein such second interface replaces or eliminates said first interface and thereby excludes said gas phase from being in contact with said liquid phase;
e) Allowing the magnetic microspheres to assemble at the second interface by ceasing to exert a magnetic force across said bottom or across said at least one wall of the incubation chamber, thereby releasing said microspheres from said bottom or from said at least one wall.

In one embodiment, steps b) and c) are performed concomitantly or in a temporally overlapping manner.

In one embodiment, said exerting a magnetic force across said closed bottom or said at least one wall of said incubation chamber in step c) occurs by either placing a magnet in vicinity to said bottom or said at least one wall of said incubation chamber or vice versa, such that by means of said magnet, a magnetic force is exerted across the bottom or said at least one wall, wherein, preferably, in step c), said bottom or said at least one wall of said incubation chamber is in physical contact with said magnet, or in such a contact that said magnet can exert a magnetic force thereacross.

In one embodiment, by step d), said incubation chamber becomes closed in that said open top is converted into a closed top by said solid surface.

In one embodiment, in step d), said solid surface is placed at said open top of said incubation chamber or at a position along said defined distance between said open top and said closed bottom of said incubation chamber, by:
- either placing only a lid at said open top of said incubation chamber or at said position,
- or placing, firstly a separate solid surface which is not a lid, e.g. a flat plate, at said open top of said incubation chamber or at said position, and thereafter placing a lid on top of said separate solid surface.

In one embodiment, said continuing to exert said magnetic force in step d) occurs by either keeping a magnet in vicinity to said bottom or said at least one wall of said incubation chamber or vice versa, such that by means of said magnet, a magnetic force continues to be exerted across the bottom or across said at least one wall, wherein, preferably, in step d), said bottom or said at least one wall of said incubation chamber is in physical contact with said magnet, or at least in such a contact that enables said magnet to exert a magnetic force thereacross.

In one embodiment, step e) is performed by removing the magnet from the vicinity of said bottom or of said at least one wall, or vice versa, thereby stopping a magnetic force from being exerted across the bottom or across said at least one wall of said incubation chamber.

In one embodiment, assembling of said microspheres at said second interface in step e) is facilitated by actively generating convection in said incubation chamber, preferably thermal convection or mechanical convection.

In one embodiment, step e) is facilitated by exposing the bottom of said incubation chamber to a temperature controlling device, preferably by thermally contacting said bottom of said incubation chamber with said temperature controlling device, more preferably by physically contacting said bottom of said incubation chamber with said temperature controlling device.

In one embodiment, said method is a method for arranging said microspheres in a layer of microspheres along said second interface, and wherein said solid surface that is placed in step d) at said open top of said incubation chamber or at said position along said defined distance between said open top and said closed bottom of said incubation chamber, is a planar solid surface.

In one embodiment, said layer is a monolayer of microspheres along said second interface.

In one embodiment, packing of microspheres in said layer is selected from random packing, regular packing, and close packing, in particular hexagonal close packing.

In one embodiment, packing of microspheres in said layer is close packing, in particular hexagonal close packing.

In one embodiment, the number of magnetic microspheres filled into said incubation chamber in step b) is chosen such it does not exceed the maximum number of microspheres that can be arranged in a layer along said second interface which layer has the closest possible packing; and/or is chosen such that the magnetic microspheres, when arranged in a layer in the closest possible packing, cover an area that is equal to or smaller than the area provided by said planar solid surface which is in contact with said liquid phase.

In one embodiment, said microspheres are mono-disperse, preferably having a coefficient of variation (CV) < 15%, more preferably a CV <10%, even more preferably a CV < 5%.

In one embodiment, said solid surface, preferably said planar solid surface comprises a region which is transparent and allows for optical probing and detection of said liquid phase and any microspheres located underneath said region.

In one embodiment, said incubation chamber is a well in a multi-well plate, and wherein said multi-well plate has a plurality of such incubation chambers, as defined in any of the foregoing claims, which are arranged in said multi-well plate, preferably arranged regularly spaced apart in said multi-well plate.

In one embodiment, said method is performed with two or more wells in said multi-well plate.

In a further aspect, the present invention also relates to a method for incubating microspheres in a non-aqueous, water-immiscible liquid phase within an incubation chamber or within a multi-well plate, said method comprising the steps:
f) performing the method for arranging microspheres according to the present invention, preferably according to an embodiment wherein said solid surface is planar and comprises a transparent region which allows for optical probing and detection;
g) performing an incubation reaction with said microspheres, wherein, optionally, said incubation reaction involves one or several changes of temperature of said microspheres.

In yet a further aspect, the present invention relates to a method for detecting and optically probing microspheres in a non-aqueous, water-immiscible liquid phase within an incubation chamber or within a multi-well plate, said method comprising the steps:
h) performing the method according to claim 16 or the method according to any of claims 17-18, when dependent on claim 16;
optionally i) performing an incubation reaction with said microspheres, wherein, optionally, said incubation reaction involves one or several changes of temperature of said microspheres;
k) detecting and optically probing said microspheres through said transparent region of said planar solid surface, preferably using a suitable detection module.

In yet another aspect, the present invention relates to a multi-well plate configured to be used in a method according to the present invention, e. g. a method for arranging microspheres, a method for incubating microspheres or a method for detecting and optically probing microspheres, said multi-well plate comprising:
- a flat body having a flat surface and comprising a plurality of wells, each well having a closed bottom, an open top, at least one wall, and a defined volume; each well being dimensioned to accommodate a defined number of magnetic microspheres or a defined volume of a suspension comprising magnetic microspheres suspended in a non-aqueous, water-immiscible liquid phase therein; wherein said wells are embedded in saidflat surface of said flat body, which flat surface is configured to be contacted by a planar solid surface, such that each well becomes closed by such planar solid surface;
- a sealing rim at the circumference of said flat body, said sealing rim surrounding said flat body of said multi-well plate and protruding therefrom;
- a lid configured to become attached to and placed on top of said multi-well plate and being dimensioned to cover and seal said multi-well plate, said lid having lower surface which is a planar solid surface; said lid having a circumference and comprising a gasket at its circumference which interacts with said sealing rim of said flat body and seals said multi-well plate, when said lid is placed on top of the multi-well plate; and
- optionally a flat plate having a lower surface which is a planar solid surface, said flat plate being configured to be placed on top of said flat body and being dimensioned to cover said flat body.

In one embodiment of the multi-well plate, each well becomes closed by a planar solid surface, such planar solid surface being provided by either said lower surface of said lid or said lower surface of said flat plate, if present.

In one embodiment of the multi-well plate, the multi-well plate further comprises a flat plate having a lower surface which is a planar solid surface, said flat plate being configured to be placed on top of said flat body and being dimensioned to cover said flat body.

In one embodiment of the multi-well plate, said lid and, if present, said flat plate, (each) comprises (comprise) a region covering the plurality of wells, which region is optically transparent and allows for optical probing and detection of any of the wells located underneath said region(s).

In one embodiment, the multi-well plate further comprises locking means configured to lock said lid in a position on top of said multi-well plate, and optionally configured to also lock said flat plate, if present, on top of said flat body of said multi-well plate, such that said multi-well plate becomes sealed.

In one embodiment of the multi-well plate, said flat body is made of or comprises a plastic, preferably a plastic having elemental carbon as an additive, more preferably a plastic having elemental carbon as an additive at a proportion of > 50 wt.%, even more preferably said plastic being polycarbonate having elemental carbon as an additive at a proportion of > 50 wt. %.

In a further aspect, the present invention also relates to a device for arranging microspheres in a non-aqueous, water-immiscible liquid phase in an incubation chamber and for excluding such microspheres from contact with or exposure to a gas phase, said device comprising:
- a receptacle to receive an incubation chamber or a multi-well plate; said receptacle having a bottom surface and at least one positioning means for positioning said incubation chamber or said multi-well plate on said bottom surface, said positioning means protruding from such bottom surface, said receptacle having dimensions to accommodate said incubation chamber or said multi-well plate in a snug fit therein; said incubation chamber having a closed bottom, an open top, at least one wall, and a defined volume; said multi-well plate being as defined herein;
- a magnet or a set of magnets located at said bottom surface of said receptacle and being incorporated therein, or located at said positioning means and being incorporated therein, said magnet or magnets being configured to exert a magnetic force across said closed bottom of said incubation chamber or across the bottom of each well in said multi-well plate, or across said at least one wall of said incubation chamber across said at least one wall of each well in said multi-well plate;
- optionally, a locking means for locking said incubation chamber or said multi-well plate in said receptacle, preferably on said bottom surface in said receptacle.

In yet another aspect, the present invention also relates to a device for incubating microspheres in a non-aqueous, water-immiscible liquid phase within an incubation chamber or within a multi-well plate, said device comprising:
- a main body having an upper surface and a receptacle configured to receive a multi-well plate as defined herein, said receptacle being located in said upper surface, said receptacle having a bottom;
- a temperature controlling device located at and/or incorporated in said bottom of said receptacle and being configured to thermally and/or physically contact the bottom of an incubation chamber or the bottom of each well of a multi-well plate, when inserted into said receptacle; and
- a lid or flap, attached to the main body and being configured to cover the upper surface of said main body and to be placed on top of said receptacle and said incubation chamber or said multi-well plate, when placed into said receptacle of said device; wherein said lid or flap is further configured to lock said incubation chamber or said multi-well plate in said receptacle when said lid or flap is placed on top of said receptacle, wherein said lid or flap furthermore comprises an optically transparent region which comes to be located on top of said receptacle and thereby allows optical probing of an incubation chamber or multi-well plate that is present in said receptacle.

In one embodiment, the device for incubating further comprises, in said receptacle, an incubation chamber or a multi-well plate as defined herein.

In yet a further aspect, the present invention relates to a device for detecting and optically probing microspheres in a non-aqueous, water-immiscible liquid phase within an incubation chamber or within a multi-well plate, said incubation chamber or said multi-well plate being located in a device for incubating as defined herein, said device for detecting comprising:
- a housing comprising a detection module, said detection module comprising an image detector, one or several filters and at least one imaging lens;
a loading bay configured to receive and accommodate a device for incubating as defined herein, said loading bay comprising an interface for electronically connecting to a device for incubating as defined herein, wherein said loading bay is located within the housing such that the detection module can be used to detect and optically probe said device for incubating when present in said loading bay, preferably can be used to detect and optically probe an incubation chamber or multi-well plate, located in said device for incubating microspheres; wherein detection and optical probing occurs through said optically transparent region of said lid or flap of said device for incubating; and wherein said optically transparent region of said lid or flap of said device for incubating is aligned with
- said optically transparent region(s) of said lid and said flat plate of said multi-well plate, if such incubation chamber or multi-well plate is present in said device for incubating,
- said image detector,
- one filter of said one or several filters, and
- said at least one imaging lens;
such that optical probing occurs through said transparent region of said lid or flap of said device for incubating and through said optically transparent region(s) of said lid and said flat plate of said multi-well plate if present in said device for detecting.

The present inventors have surprisingly found that it is possible to arrange microspheres in a non-aqueous, water-immiscible liquid phase in an incubation chamber and to prevent such microspheres from becoming contacted with or exposed to a gas phase. In accordance with embodiments of the present invention this is achieved by making use of buoyancy differences, or density differences, of the microspheres and the liquid phase in which they are suspended, and by endowing such microspheres with magnetic qualities (i. e. by making them magnetic), and using such magnetic qualities to handle the microspheres within the incubation chamber, for example by attracting them to the bottom or a wall of an incubation chamber, whilst, at the same time, a solid surface (e.g. of a substrate) is placed at an open top of the incubation chamber such that the solid surface comes into contact with the liquid phase whereby a gas phase (that was previously in contact with the liquid phase) is excluded from being in contact with the liquid phase. In doing so, any interface between said liquid phase and a gas phase surrounding the incubation chamber is removed or excluded and is replaced by another interface which is an interface between the solid surface and the liquid phase.

Examples of microspheres that are suitable to be used in the context of the present invention are e.g. microspheres as described in previous patent applications by the present patent applicant, e. g. as described in European patent application No. 21 206 745.8, filed on November 5, 2021; or as described in International patent application No. PCT/EP2017/084370 (published as WO 2018/122162), filed on December 22, 2017, or as described in International patent applications No. PCT/EP2020/086171 and PCT/EP2020/086194 (published as WO 2021/122563 and WO 2021/122579, respectively). Broadly speaking, microspheres, in accordance with the present invention are microspheres comprising a hydrogel and including a volume for receiving an aqueous solution and for providing a reaction space in which a suitable chemical or biochemical reaction may take place.

The term "microsphere", as used herein, is intended to refer to spherical bodies, or substantially spherical or ellipsoidal or otherwise regularly round-shaped, e. g. egg-shaped bodies, which comprise a hydrogel and provide for a reaction space within their volume. Typically, "microspheres" in accordance with the present invention have an average diameter, preferably, along their longest extension, in the range of from 20 µm to 500 µm, preferably from 20 µm to 200 µm, more preferably from 20 µm to 100 µm.

Such microspheres can be made magnetic by incorporating smaller magnetic particles into such microspheres. Incorporation of such magnetic particles into microspheres allows such microspheres to be attracted by a magnet (permanent or electrical) placed in vicinity to the microspheres. The term "magnetic particles" as used herein, is meant to refer to particles, showing in essence non-diamagnetic behavior, thus allowing such particles to be attracted by a magnet. In one embodiment, such magnetic particles are ferromagnetic particles. In another embodiment, such magnetic particles are paramagnetic particles. In another embodiment, such magnetic particles are ferrimagnetic particles. In yet another embodiment, such magnetic particles are super-paramagnetic particles. In a preferred embodiment, such magnetic particles are ferromagnetic or superparamagnetic particles. The term "magnetic particles", as used herein, is meant to exclude any particles with diamagnetic behavior. "Magnetic particles", as used herein, preferably, have a size and average diameter or an average long extension in one dimension, that is small enough for such magnetic particles to be incorporated into microspheres according to the present invention. In embodiments of the present invention, such magnetic particles have a size and average diameter or an average long extension in one dimension, that is in the range of from 50 nm to 10 µm, preferably 100 nm to 5 µm, more preferably 1 µm to 5 µm, even more preferably 1 µm to 3 µm. Preferably, the size and average diameter or average long extension in one dimension of the magnetic particles is chosen such that it can be easily accommodated within microspheres according to the present invention. In many instances, microspheres in accordance with embodiments of the present invention may comprise a hydrogel that forms a porous network, and for such network, the size and average diameter or average long extension in one dimension of the magnetic particles is chosen such that it is larger than the average mesh or pore size of the network of the micropsheres.

In accordance with embodiments of the present invention, such magnetic microspheres may be filled with appropriate reagents for performing a chemical or biochemical reaction, and such reagents will be incorporated as an aqueous mixture into the volume(s) of said microspheres and will be contained thereby. Subsequently they are suspended in a non-aqueous, water-immiscible liquid phase, and are filled, as suspension, into an incubation chamber having a closed bottom, an open top, at least one wall, which may, for example, be a circumferential wall, and a defined volume. If the incubation chamber has one wall this may be a circumferential wall, thus making the incubation chamber having a round(ed) cross-section. If it has several walls, the respective cross-section will be triangular, square, rectangular etc. depending on the number of walls of said incubation chamber.

The non-aqueous, water-immiscible liquid phase is chosen such that it has a density that is larger than the density of the magnetic microspheres, including any aqueous mixture contained in the microspheres. To put it differently, it is preferred that the magnetic microspheres, including any aqueous mixture contained in the microspheres, will have a first defined density which is smaller than the density of the non-aqueous, water-immiscible liquid phase, i. e. the "second defined density". The smaller density of the microspheres will result in a buoyancy of the microspheres within the non-aqueous, water-immiscible liquid phase. Examples of a suitable non-aqueous, water-immiscible liquid phase are oils, in particular mineral oils, fluorocarbon (FC) oils, perfluorocarbon (PFC) oils, perfluoropolyether (PFPE) oils, and hydrofluoroether (HFE) oils. An example of a suitable hydrofluoroether oil is Novec 7500^{®}, commercially available for example from 3M Deutschland GmbH, Neuss, Germany. In some embodiments, the non-aqueous, water-immiscible liquid phase may be or is supplemented with additives such as an emulsifier, a surfactants, and/or a stabilizer etc.

Subsequently, in accordance with embodiments of the method according to the present invention, the suspension of microspheres is filled into the incubation chamber, as a result of which there is formed a first interface between the non-aqueous, water-immiscible liquid phase and a gas phase which surrounds the incubation chamber. Such first interface is formed at the open top or at a first position along said defined distance between said open top and said closed bottom. The position where such first interface is formed depends on whether in step b) said incubation chamber is filled, with said suspension of magnetic microspheres in a non-aqueous, water-immiscible liquid phase, completely or only partially. If the incubation chamber is filled completely, i.e. up to its open top, then the first interface will be formed at the open top, If the incubation chamber is filled only partially, i.e. not up to its open top, then the first interface will be formed at a first position along said defined distance between said open top and said closed bottom. The precise location of such first position depends on the amount of suspension that is filled into said incubation chamber. Typically, however, such first position will be located within the incubation chamber and will therefore be below said open top of said incubation chamber. Such first position therefore will be different from the open top of said incubation chamber.

In the absence of a magnet in vicinity to the incubation chamber/the magnetic microspheres located in such incubation chamber, these microspheres will tend to move towards the first interface between the non-aqueous, water-immiscible liquid phase and the gas phase surrounding the incubation chamber. As a result, magnetic microspheres will become at least partially exposed to said gas phase. If, however, a magnetic force is exerted across the closed bottom of the incubation chamber or across a wall of the incubation chamber, the magnetic microspheres will be attracted thereto and can be kept attracted thereto as long as such magnetic force is exerted. Effectively, they will therefore be removed from said first interface, and will be fully submerged in said non-aqueous water-immiscible liquid phase. In accordance with embodiments of the present invention, whilst the magnetic microspheres are kept attracted to the bottom or the wall(s) of the incubation chamber, subsequently a solid surface is placed at the open top of the incubation chamber or at a position along a defined distance between the open top and the closed bottom of the incubation chamber, wherein such placing occurs such that the solid surface comes in contact with the liquid phase and such that at said open top or at said position along the defined distance between the open top and the closed bottom of the incubation chamber, there is formed a second interface which is an interface between the non-aqueous, water-immiscible liquid phase and the solid surface. As a result of such placement, such second interface replaces or eliminates the first interface (between the non-aqueous, water-immiscible liquid phase and the gas phase surrounding the incubation chamber) and thereby excludes the gas phase from being in contact with the liquid phase and, therefore also from being in contact with the microspheres. Typically, the position where said solid surface is placed in step d), depends on the location of the first interface, i.e. the interface between said non-aqueous, water-immiscible liquid phase and the gas phase surrounding the incubation chamber. If the first interface is located at the open top of said incubation chamber (because the incubation chamber has been filled in step b) completely up to its open top, with said suspension), then in step d) the solid surface is placed at said open top. If, however, the first interface is located at a first position within the incubation chamber which first position is different from said open top (because the incubation chamber has been filled in step b) only partially with said suspension), then in step d) said solid surface is preferably placed either at a position that matches the first position, namely the location of said first interface, or that is located below the first position of the first interface. Hence, in some embodiments, such position where the solid surface is placed in step d), may be the first position. Alternatively, the solid surface may also be placed in step d) at a position that is below the first position within the incubation chamber, i.e. below the location of the first interface, so as to absolutely ensure that the second interface that is formed, replaces or eliminates the first interface. Irrespective of the position at which the solid surface is placed in step d), step d) should be performed in such a manner that, as a result of such step d), said solid surface is in contact with said liquid phase and such that at said open top or at said position where the solid surface is placed in step d), there is formed a second interface between said non-aqueous, water-immiscible liquid phase and said solid surface, wherein such second interface replaces or eliminates said first interface and thereby excludes said gas phase from being in contact with said liquid phase;

In accordance with embodiments of the present invention, the solid surface that is placed at the open top of the incubation chamber or at a position along a defined distance between the open top and the closed bottom of the incubation chamber, is chosen such in its dimensions that it will completely cover said liquid phase and/or said open top of said incubation chamber, so as to form a second interface that replaces or eliminates the first interface (between the non-aqueous, water-immiscible liquid phase and the gas phase surrounding the incubation chamber). More specifically, in some embodiments, the solid surface placed at the open top of the incubation chamber or at a position along a defined distance between the open top and the closed bottom of the incubation chamber, is chosen such in its dimensions that it will completely cover said liquid phase and/or said open top of said incubation chamber, and has a cross-section that allows the formation of a second interface between the non-aqueous, water-immiscible liquid phase and said solid surface which second interface replaces or eliminates the first interface and thereby excludes said gas phase (surrounding the incubation chamber) from being in contact with said non-aqueous, water-immiscible liquid phase. In a particularly preferred embodiment, the cross-section of said solid surface is chosen such that it allows to insert said solid surface into said incubation chamber and/or snugly fit with the wall(s) of said incubation chamber.

In some embodiments, where the incubation chamber is a well in a multi-well plate, preferably, the first interface is located at the open top of each respective well (because, preferably, each well has been filled in step b) completely up to its open top, with said suspension). In such embodiments, in step d) the solid surface is placed at said open top of each respective well. In some such embodiments, said solid surface may be provided separately for each well, or, alternatively, may be provided as a single solid surface for all wells together. In these embodiments, it is preferred that said solid surface is placed at said open top(s) of said respective well(s) by
- either placing only a lid of said multi-well plate at said open top(s) of said respective well(s),
- or placing, firstly a separate solid surface which is not a lid, e.g. a flat plate, at said open top(s) of said respective well(s), and thereafter placing a lid of said multi-well plate on top of said separate solid surface.

Finally, in accordance with embodiments of the present invention in step e), the magnetic microspheres are allowed to assemble at the second interface by ceasing to exert a magnetic force across the bottom of the incubation chamber or across a wall of the incubation chamber, thereby releasing the microspheres therefrom and allowing them to assemble at the second interface, because of their lower density in comparison to the density of the non-aqueous, water-immiscible liquid phase.

In accordance with embodiments of the present invention, the term "magnet" is meant to refer to any material or object or body that produces a magnetic field that is capable of exerting a magnetic force on magnetic materials. Magnets in accordance with embodiments of the present invention may be permanent magnets, such as ferromagnetic materials, or they may be electromagnets which can be switched on and off, thus enabling an easy performance of steps c) and d) in which a magnetic force is exerted, and of step e) in which exertion of a magnetic force is ceased.

It should be noted that depending on the shape, form, dimensions and other qualities of the solid surface that is placed at the open top of the incubation chamber or at a position along the defined distance between the open top and the closed bottom of the incubation chamber in step d), and that forms the second interface, the microspheres may be arranged accordingly. For protocols involving a simple incubation of microspheres, for example for performing a nucleic acid amplification in such microspheres, without subsequent detection, it is sufficient if the microspheres in step c) become simply removed from the first interface and become thereby excluded from a gas phase surrounding the incubation chamber (or well within a multi-well plate). In such embodiment, it is sufficient if the solid surface that is placed in step d), is a surface of any shape and dimensions as long as such surface removes the microspheres from the gas phase, i. e. the first interface, and prevents them from coming into contact therewith, by forming a second interface that replaces or eliminates the first interface. For example, a curved solid surface of suitable dimensions is perfectly fine for such purpose. If, however, the microspheres are used in a protocol which also involves not only a nucleic acid amplification but also a simultaneous or subsequent detection of a signal generated within the microspheres and/or imaging of such signal, it is important that these microspheres become arranged in a layer, preferably, a monolayer, that preferably allows for an optical probing, imaging and/or optical investigation of said microspheres. Such arrangement in a layer, preferably a monolayer can be achieved by a solid surface that is a planar solid surface.

In one embodiment, the method according to the invention is a method for arranging the microspheres in a layer of microspheres along the second interface. In a preferred embodiment, such layer is a monolayer of microspheres along the second interface. When the microspheres are arranged in a layer or monolayer in accordance with embodiments of the present invention, their packing may be selected and tailored appropriately. In one embodiment, the packing of microspheres in such layer or monolayer is selected from random packing, regular packing and close packing, in particular hexagonal close packing. In a preferred embodiment, the packing of microspheres in the layer or monolayer is close packing, more preferably hexagonal close packing.

Especially in those embodiments, where the microspheres become arranged in a layer or monolayer of microspheres along the second interface, it is preferred that the solid surface that is placed at the open top of the incubation chamber or at a second position along the defined distance between the open top and the closed bottom of the incubation chamber in step d), is a planar solid surface. Especially in those embodiments involving a planar solid surface, the arrangement of the magnetic microspheres into a layer, preferably, a monolayer, can be achieved.

The term "planar surface" or "planar solid surface", as used herein, is meant to refer to a surface which is essentially flat and the appearance of which is or approximates a plane. In some embodiments, such planar solid surface may be a smooth surface. Such term "planar surface" or "planar solid surface" should however also be interpreted as still allowing for a certain degree of surface roughness in such plane. For the sake of clarity "planar surface" or "planar solid surface" may also be a surface that may include socertain structures designed to facilitate a specific packing of said microspheres in said layer or monolayer that forms along said planar surface (and second interface). Also, for example, such "planar surface" or "planar solid surface" may have a certain degree of patterning, e.g. for achieving or facilitating a specific packing of said microspheres in said layer or monolayer

Apart from the influence that the planar solid surface (and possible structures or patterns thereon) may have on the packing of said microspheres, the packing of microspheres in a layer arrangement can furthermore also be tailored by choosing an appropriate concentration of microspheres within the non-aqueous, water-immiscible liquid phase in relation to the area/size of the solid planar surface, and/or by choosing the total number of magnetic microspheres filled into the incubation chamber in step b) in relation to the area/size of the solid planar surface. In one embodiment, the number of magnetic microspheres filled into the incubation chamber in step b) is chosen such that it is below, preferably significantly below, the maximum number of microspheres that can be arranged in a layer along the second interface, which layer would have the closest possible packing. This will result in a random packing or regular packing of microspheres in such layer. In another embodiment, the number of magnetic microspheres filled into the incubation chamber in step b) is chosen such that it does not exceed the maximum number of microspheres that can be arranged in a layer along the second interface which layer would have the closest possible packing, but which approaches such maximum number or is nearly equal to such maximum number or is, in fact, the maximum number of microspheres that can (theoretically) be arranged in a layer along the second interface. This will result in a close packing or even hexagonal close packing of microspheres in such layer. In one embodiment, the number of magnetic microspheres filled into the incubation chamber in step b) is chosen such that the magnetic microspheres, when arranged in a layer in a closest possible packing, cover an area that is equal to or smaller than the area provided by said solid surface which is in contact with the liquid phase.

If, on the other hand, the number of magnetic microspheres filled into the incubation chamber in step b) is chosen such that it does exceed the maximum number of microspheres that can be arranged in a layer along the second interface which layer would have the closest possible packing, then there will not be a simple layer of microspheres, in particular not a monolayer of microspheres, arranged along the second interface, but rather the microspheres will arrange in a three-dimensional irregular bulk volume. This is, because such (high or excessive) number of microspheres can only be arranged along the second interface by accommodating such (high or excessive) number of microspheres in a three-dimensional bulk volume, rather than in a single layer or, more precisely a monolayer of microspheres.

In another aspect, the present invention also relates to a multi-well plate configured to be used in a method according to embodiments of the present invention as defined herein. In accordance with embodiments of the present invention, such multi-well plate comprises:
- A flat body having a flat surface and comprising a plurality of wells, each well having a closed bottom, an open top, at least one wall, and a defined volume; each well being dimensioned to accommodate a defined number of magnetic microspheres or a defined volume of a suspension comprising magnetic microspheres suspended in a non-aqueous-water-immiscible liquid phase therein; wherein said wells are embedded in said flat surface of said flat body, which flat surface is configured to be contacted by a solid surface, preferably a planar solid surface, such that each well becomes closed by such solid surface, preferably by such planar solid surface;
- a sealing rim at the circumference of said flat body, said sealing rim surrounding said flat body of said multi-well plate and protruding therefrom;
- a lid configured to become attached to and placed on top of said multi-well plate and being dimensioned to cover and seal said multi-well plate, said lid having a lower surface which is a solid surface, preferably a planar solid surface, said lid having a circumference and comprising a gasket at its circumference which interacts with said sealing rim of said flat body and seals said multi-well plate, when said lid is placed on top of the multi-well plate; and
- optionally a flat plate having a lower surface which is a planar solid surface, said flat plate being configured to be placed on top of said flat body and being dimensioned to cover said flat body.

It should be noted that in embodiments, where a flat plate having a lower surface, is present, it is typically placed on top of the flat body and is dimensioned to cover said flat body, and said lid is configured to be placed on top of the multi-well plate by being placed on top of such flat plate. In a preferred embodiment, the flat plate that is placed on top of the flat body of the multi-well plate has a sufficient density/weight that, under the influence of gravity, allows it to rest on top of the flat body of the multi-well plate and to remain there even without being contacted and fixed by the lid of said multi-well plate that is placed on top of the multi-well plate. For example, such flat plate may be made of a material endowing it with sufficient density/weight and allowing it to rest on top of the flat body of the multi-well plate and to remain there even without being contacted and without being fixed by the lid of said multi-well plate. An example of such suitable material for the flat plate is glass or quartz. In such embodiment, even though the lid is placed on top of said multi-well plate and also effectively also on top of said flat plate, it does not need to contact, and indeed does not contact, the flat plate in order for such flat plate to rest and remain on top of the flat body of the multi-well plate.

In other embodiments, however, the lid is configured to be placed on top of the multi-well plate by actually contacting the flat plate on top thereof, such that the flat plate rests on the flat body of the multi-well plate and is in contact therewith, and furthermore is contacted (on its other side) by the lid that is placed on top of the flat plate. In such embodiments, the flat plate, on one side, is effectively contacted by the lid and, on the other side, itself contacts the flat body of the multi-well plate. In such embodiments, the lid also helps to fix the flat plate on the flat body of the multi-well plate, by contacting the flat plate.

The multi-well plate in accordance with embodiments of the present invention is particularly suitable for arranging microspheres in a non-aqueous, water-immiscible liquid phase in the wells of said multi-well plate and for excluding such microspheres from contact with or exposure to a gas phase.

In a further aspect, the present invention relates to a device for arranging microspheres in a non-aqueous, water-immiscible liquid phase in some or all of the wells of a multi-well plate in accordance with the present invention, and for excluding such microspheres from contact with or exposure to a gas phase.

In accordance with embodiments of the present invention, such device comprises:
- A receptacle to receive an incubation chamber or a multi-well plate; said receptacle having a bottom surface and at least one positioning means for positioning said incubation chamber or said multi-well plate on said bottom surface, said positioning means protruding from such bottom surface, and having dimensions to accommodate said incubation chamber or said multi-well plate in a snug fit in said receptacle; said incubation chamber having a closed bottom, an open top, at least one wall and a defined volume; said multi-well plate being as defined in embodiments herein;
- a magnet or a set of magnets located at said bottom surface of said receptacle and being incorporated therein, or located at said positioning means and being incorporated therein, said magnet or magnets being configured to exert a magnetic force across said closed bottom of said incubation chamber or across the bottom of each well in said multi-well plate, or across said at least one wall of said incubation chamber or across said at least one wall of each well in said multi-well plate;
- optionally, a locking means for locking, preferably reversibly locking, said incubation chamber or said multi-well plate in said receptacle, preferably on said bottom surface in said receptacle.

Furthermore, in another aspect, the present invention relates to a device for incubating microspheres in a non-aqueous, water-immiscible liquid phase within an incubation chamber or within a multi-well plate, said device comprising:
- a main body having an upper surface and a receptacle configured to receive a multi-well plate in accordance with embodiments of the present invention as defined herein, said receptacle being located in said upper surface, said receptacle having a bottom;
- a temperature controlling device located at and/or incorporated in said bottom of said receptacle and being configured to thermally and/or physically contact the bottom of an incubation chamber or the bottom of each well of a multi-well plate, when inserted into said receptacle; and
- a lid or flap, attached to the main body and being configured to cover the upper surface of said main body and to be placed on top of said receptacle and said incubation chamber or said multi-well plate, when placed into said receptacle of said device; wherein said lid or flap is further configured to lock said incubation chamber or said multi-well plate in said receptacle when said lid or flap is placed on top of said receptacle, wherein said lid or flap furthermore comprises an optically transparent region which comes to be located on top of said receptacle and thereby allows optical probing of an incubation chamber or multi-well plate that is present in said receptacle.

In one embodiment, the device for incubating microspheres in a non-aqueous, water-immiscible liquid phase further comprises, in said receptacle, an incubation chamber or a multi-well plate according to the present invention, as defined herein.

In yet another aspect, the present invention relates to a device for detecting and optically probing microspheres in a non-aqueous, water-immiscible liquid phase within an incubation chamber or within a multi-well plate, said incubation chamber or said multi-well plate being located in a device for incubating according to the present invention, as defined herein, said device for detecting comprising:
- a housing comprising a detection module, said detection module comprising an image detector, one or several filters and at least one imaging lens;
- a loading bay configured to receive and accommodate a device for incubating according to the present invention, as defined herein, said loading bay comprising an interface for electronically connecting to a device for incubating according to the present invention, as defined herein, wherein said loading bay is located within the housing such that the detection module can be used to detect and optically probe said device for incubating when present in said loading bay, preferably can be used to detect and optically probe an incubation chamber or multi-well plate, located in said device for incubating microspheres; wherein detection and optical probing occurs through said optically transparent region of said lid or flap of said device for incubating; and wherein said optically transparent region of said lid or flap of said device for incubating is aligned with
   - said optically transparent region(s) of said lid and of said flat plate of said multi-well plate, if such incubation chamber or multi-well plate is present in said device for incubating,
   - said image detector,
   - one filter of said one or several filters, and
   - said at least one imaging lens;
   such that optical probing occurs through said transparent region of said lid or flap of said device for incubating and through said optically transparent region(s) of said lid and said flat plate of said multi-well plate, if present in said device for detecting.

In yet another aspect, the present invention also relates to the use of elemental carbon, in particular of graphite, for incorporating into a plastic material to increase the heat conductivity of such plastic material and to thereby make such plastic material particularly suitable for manufacturing an incubation chamber or a multi-well plate for performing an incubation reaction, such as a nucleic acid amplification reaction, involving one or several changes of temperature.

In yet another aspect, the present invention relates to the use of elemental carbon, in particular graphite, for increasing heat conductivity of an incubation chamber or of a multi-well plate, wherein such use involves the incorporation of said elemental carbon, e. g. graphite, into the plastic material from which said incubation chamber or said multi-well plate is made. In particularly preferred embodiments of such use(s), the plastic material from which the incubation chamber and/or multi-well plate is made, is polycarbonate.

In a further aspect, the present invention relates to the use of elemental carbon, in particular graphite, for manufacturing an incubation chamber or a multi-well plate that is configured for performing an incubation reaction, such as a nucleic acid amplification reaction, involving one or several changes of temperature of an incubated sample, wherein such manufacturing includes the incorporation of said elemental carbon into the plastic material from which said incubation chamber or said multi-well plate is made. In one embodiment of such use, the incubation chamber or multi-well plate, by virtue of the incorporation of said elemental carbon into the plastic material, is configured to perform an incubation reaction involving one or several changes of temperature of an incubated sample in accelerated manner, in comparison to the same incubation reaction when performed in an incubation chamber or multi-well plate into the plastic material of which no elemental carbon, e.g. graphite, has been incorporated.

In one embodiment of such use, the incubation chamber or multi-well plate, that is configured for performing an incubation reaction, such as a nucleic acid amplification reaction, involving one or several changes of temperature of an incubated sample, is configured to perform such incubation reaction in a manner that involves one or several changes of temperature of an incubated sample with a temperature difference of 20°C to 25°C achieved over a time period < 5s per individual change of temperature, preferably with a temperature difference of 20°C to 25°C achieved over a time period < 3.5s per individual change of temperature.

In a further aspect, the present invention also relates to an incubation chamber or a multi-well plate for performing an incubation reaction, such as a nucleic acid amplification reaction, involving one or several changes of temperature, which incubation chamber or multi-well plate is made of plastic into which elemental carbon, in particular graphite, has been incorporated. In a preferred embodiment of this aspect, the plastic material from which the incubation chamber and/or multi-well plate is made, is polycarbonate. Such incubation chamber or multi-well plate in accordance with this aspect of the present invention is configured to perform an incubation reaction involving one or several changes of temperature of an incubated sample in accelerated manner, in comparison to the same incubation reaction when performed in an incubation chamber or multi-well plate into the plastic material of which no elemental carbon, e.g. graphite, has been incorporated.

An example of suitable plastic material in which elemental carbon has been incorporated is "Makrolon TC621^{®}", which is commercially available from Covestro AG and which may be used in this aspect of the present invention.

Moreover, reference is made to the figures, wherein:

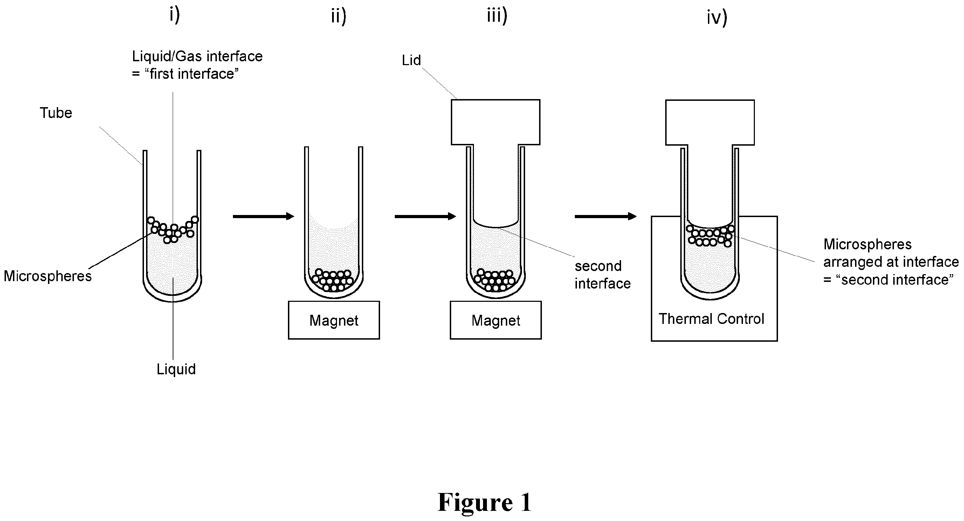

Figure 1 shows a schematic flow diagram of an embodiment of a method for arranging microspheres in a non-aqueous, water-immiscible liquid phase in a single incubation chamber and for excluding such microspheres from contact with or exposure to a gas phase. The microspheres initially are located at an interface between the non-aqueous, water-immiscible liquid phase and the gas phase surrounding the incubation chamber. Thereafter the microspheres are attracted to the bottom by means of a magnet and a solid surface is placed at the top of the incubation chamber or at a position within the incubation chamber, namely at a position along a defined distance between the open top and the closed bottom of the incubation chamber, such that the solid surface comes into contact with the liquid phase and such that at the open top of the incubation chamber or at the respective position within the incubation chamber where the solid surface is placed, there is formed another interface between the non-aqueous water-immiscible liquid phase and the solid surface, and there is no longer an interface between the liquid phase and the gas phase, which interface therefore has been replaced or eliminated by the other interface between the solid surface and the liquid phase. Thereafter, the microspheres are released by removing the magnet. The incubation chamber is then placed under thermal control for subsequent reactions. It should be noted that in this figure the solid surface is or may be a curved surface as a result of which arrangement of the microspheres occurs in an irregular fashion, rather than a layer. However, also with such surface an interface with the surrounding gas phase, e.g. air is excluded.
   The letters in figure 1 correspond to the respective steps of the method for arranging microspheres according to the present invention as follows:
   Letter i) of figure 1 corresponds to a combination of steps a) and b) of the method for arranging microspheres according to the present invention. Letter ii) of figure 1 corresponds to step c) of the method for arranging microspheres according to the present invention and letter iii) of figure 1 corresponds to step d) of the method for arranging microspheres according to the present invention. Letter iv) of figure 1 corresponds to step e) of the method according to the present invention, with the additional limitation that in figure 1, an additional (optional) thermal control element is shown. It is clear to a person skilled in the art, that the method for arranging microspheres according to the present invention, however, also works without such thermal control. In some instances, however, such thermal control may be preferred, because it allows for additional convection and therefore facilitates an arrangement of the microspheres along the second interface.

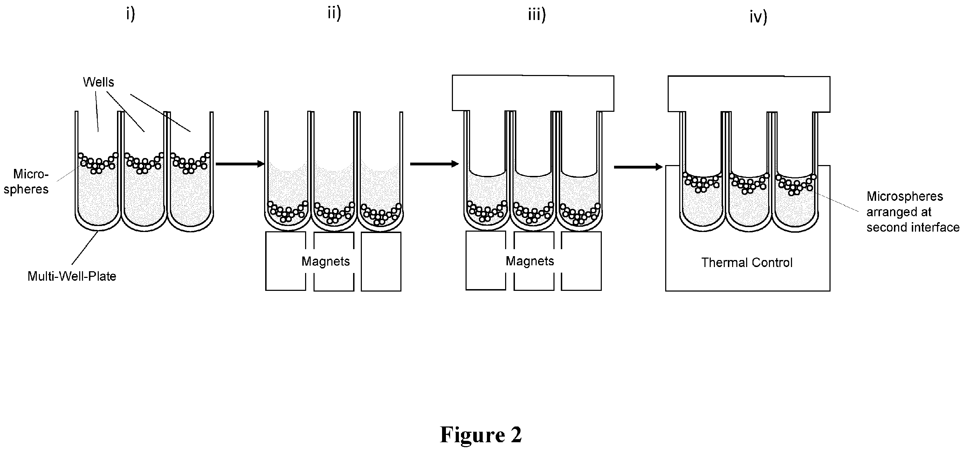

Figure 2 shows the same schematic flow diagram of an embodiment of a method for arranging microspheres in a non-aqueous, water-immiscible liquid phase and for excluding such microspheres from contact with or exposure to a gas phase, but this time for a multiplicity of incubation chambers, such as are, for example encountered in a multi-well plate, i.e. a plurality of wells. The respective letters in this figure 2 are as for figure 1.

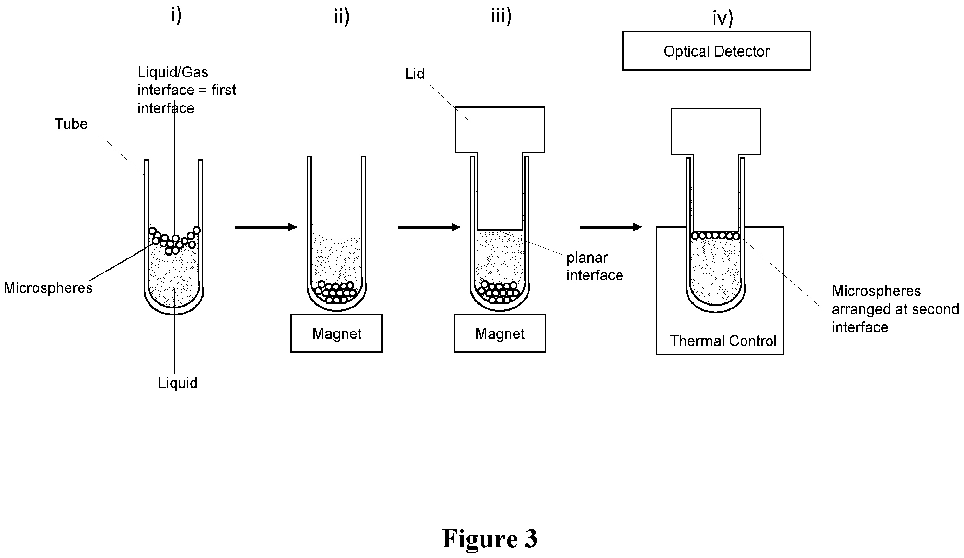

Figure 3 shows a schematic flow diagram of an embodiment of a method for arranging microspheres in a non-aqueous, water-immiscible liquid phase in an incubation chamber and for excluding such microspheres from contact with or exposure to a gas phase, wherein the method employs a solid surface that is placed on top of the incubation chamber or at a position within the incubation chamber, namely at a position along a defined distance between the open top and the closed bottom of the incubation chamber, wherein, however, in contrast to figures 2 and 3, the solid surface employed here is a planar solid surface. This allows the microspheres to be arranged in a plane layer along such planar solid surface. The planar solid surface may for example be the lid of the incubation chamber. Such embodiment is particularly of interest, where the microspheres, after having been arranged and after having undergone a biochemical reaction, such as an amplification reaction, are to be optically probed and further investigated. This is additionally shown in figure 3 letter iv), by a schematic (optional) optical detector. Such optional optical detector is, however, not necessarily part of a method for arranging in accordance with the present invention. Such optical detector is a part of a method for detecting and optically probing in accordance with the present invention.

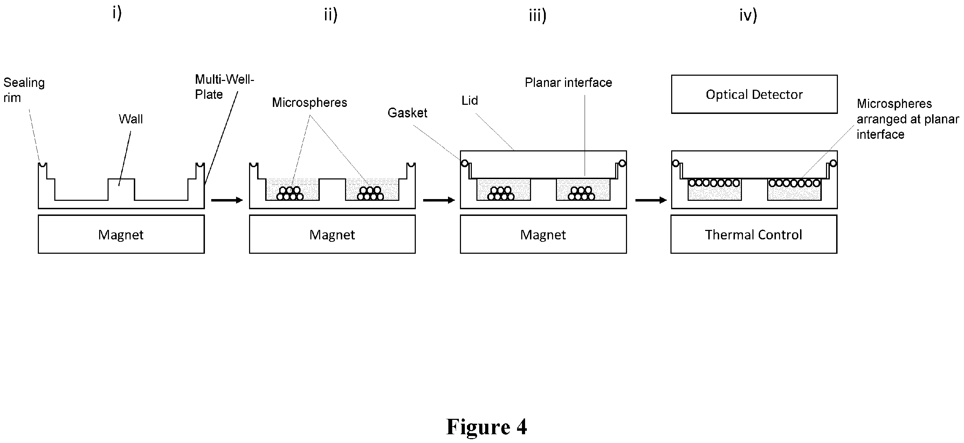

Figure 4 shows a schematic flow diagram of an embodiment of a method for arranging microspheres in a non-aqueous, water-immiscible liquid phase and for excluding such microspheres from contact with or exposure to a gas phase, this time in a plurality of incubation chambers, such as are for example found as wells in a multi-well plate. As with figure 3, the method employs a planar solid surface that forms a second interface, along which the microspheres can be arranged, allowing arrangement thereof in a layer arrangement and thus facilitating subsequent optical probing and investigation. The solid surface may for example be a lid of the multi-well plate.

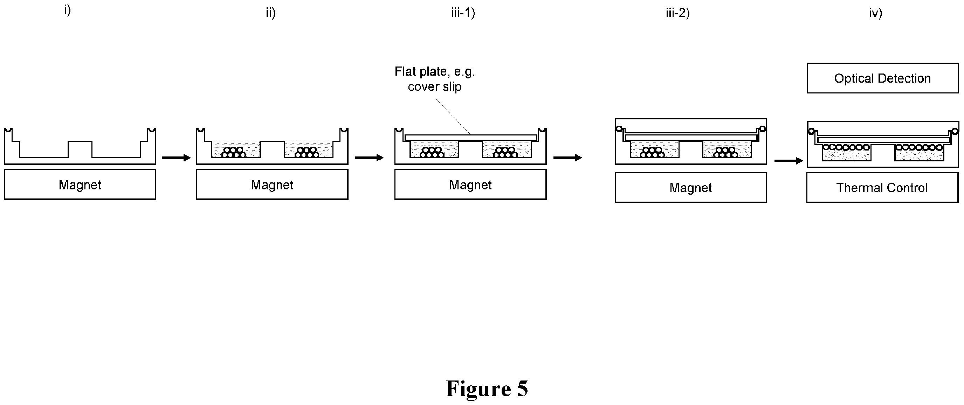

Figure 5 is similar to an embodiment as shown in figure 4, except for that this embodiment additionally employs a separate solid planar surface which is separate from the lid, which separate solid planar surface functions as a planar solid surface to form the second interface at which the microspheres are arranged. The separate solid planar surface maybe a flat plate, e.g. a cover slip. Such embodiment is particularly useful, in that it allows the decoupling of a functionality for sealing the multi-well plate which is achieved by a lid in figure 5, letter iii-2), and the functionality of providing an interface for arrangement of microspheres along such interface, as can be seen in in figure 5, letter iii-1). This allows for broad manufacturing tolerances and thus for a lower disposable costs which by far compensates for the additional part, i. e. the additional flat plate, e. g. the cover slip.

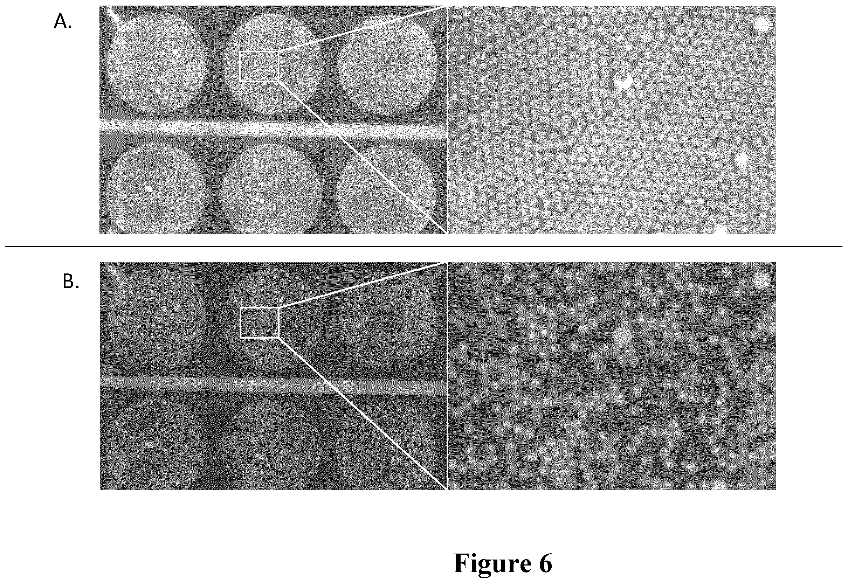

Figure 6 shows fluorescence image of an embodiment of a multi-well plate equipped with microspheres, wherein the microspheres form a layer that is closely packed.
   More specifically, Figure 6 shows fluorescence images of a MiniWell Plate (i.e. a specific multi-well plate having 6 wells only) equipped with microspheres representing "nanoreactor beads". Imaging has been performed following thermocycling according to the following thermal protocol:
   - 10 min 50°C (reverse transcriptase step)
   - 2 min 80°C (system preheating)
   - 2 min 98°C (initial denaturation)
   - 40 cycles with 30s 98°C & 30s 57°C
Figure 6A:
   Fluorescence image acquired with FAM fluorescence filter set of microspheres ("nanoreactor beads") processed on a mini-well-plate with six wells. All beads are stained with FAM dye for bead recognition and automatic image segmentation. The wells are densely packed with nanoreactor beads. The enlarged view of the marked area shows a self-assembled monolayer of hexagonally packed nanoreactor beads.
Figure 6B:
   The same beads as shown in A. have been equipped with a set of primers specific for a target in MS2 Phage RNA. In addition, a TaqMan probe labelled with Cy5 dye has been used to provide for detection of target specific amplification of target present in some of the beads. An overview fluorescence image of a mini well plate with six wells acquired with Cy5 fluorescence filter set is shown on the left. The enlarged view of the labelled area on the right shows densely packed dark and bright nanoreactor beads. The bright signal is generated by amplicon formation during thermocycling and indicate the presence of target on the respective nanoreactor bead.

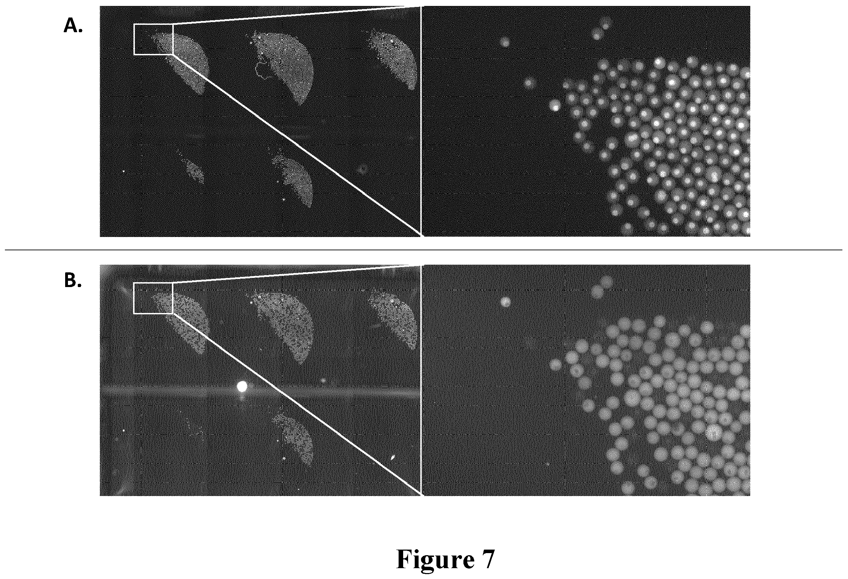

Figure 7 shows an arrangement of microspheres in a layer, wherein, however, packing is random packing, and there are defects in the layer/monolayer of microspheres. More specifically, figure 7 shows fluorescence images of a Mini-Well-Plate equipped with microspheres representing "nanoreactor beads". Imaging has been performed following thermocycling of according to the following thermal protocol:
   - 2 min 98°C (initial denaturation)
   - 45 cycles with 5s 98°C & 15s 59°C
Figure 7A:
   Fluorescence image acquired with Cy5 fluorescence filter set. All microspheres (="beads" or "nanoreactor beads") are stained with Cy5 dye for bead recognition and automatic image segmentation. The wells are only partly occupied by the nanoreactor beads. Despite that the enlarged view of the marked area shows a monolayer of nanoreactor beads.
Figure 7B:
   FAM-Channel:
   Signal of FAM- TaqMan probe with genomic DNA as a template and RPP30 as target
   The same beads as shown in A., equipped with a set of primers specific for a target represented in the human RPP30 gene. The beads also contained a target specific TaqMan probe labelled with FAM dye. An overview fluorescence image of the mini well plate with six wells acquired with FAM fluorescence filter set is shown on the left. The enlarged view of the labelled area on the right shows the same beads dark and bright, respectively. The bright signal here indicates PCR amplification of the molecular target.

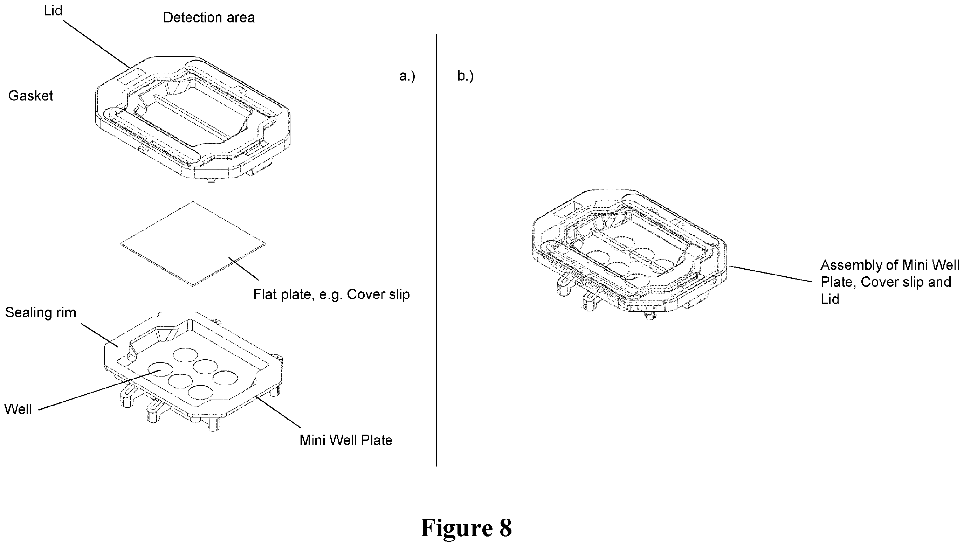

Figure 8 a) shows an explosion view of a of an embodiment of a disassembled multi-well plate, referred to in the figure as a "Mini-Well-Plate" with a plurality of wells, e.g. 6 wells, a sealing rim, and a separate flat plate, e.g. a cover slip, as a well as a lid. The lid has a gasket allowing for the sealing of the well plate and also encompasses a detection area which is optically transmissible, as is the flat plate cover slip, in order to allow for a subsequent optical detection. Figure 8b) is an isometric view of a closed assembly of the multi-well plate of a) including the flat plate (= cover slip) and the lid.

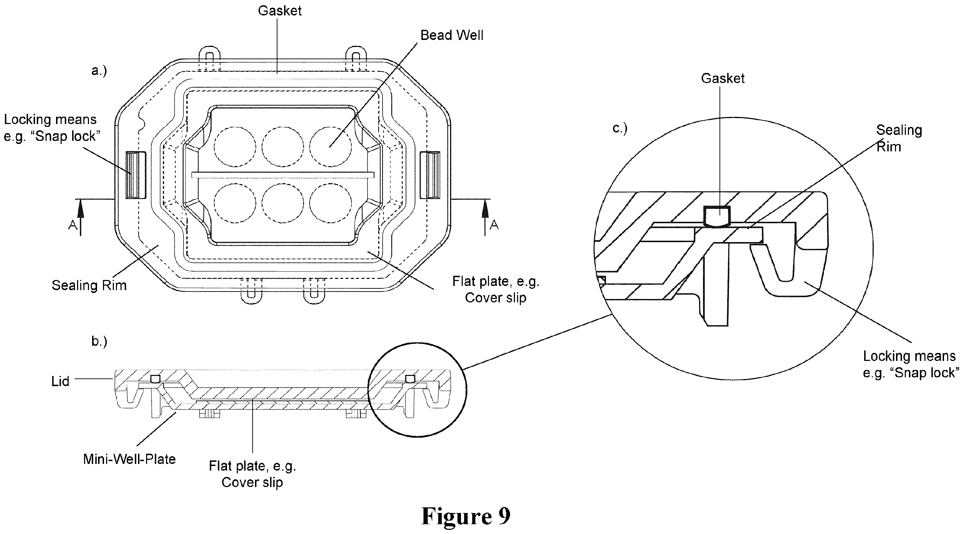

Figure 9 a) shows a top view of an of an embodiment of an assembly of a multi-well plate, flat plate, e.g. cover slip and lid. Line A indicates the cut surface for the cross section shown in figure 9b). Also shown in figure 9b) is a detail on the right that is encircled and is shown in greater detail in figure 9c) which shows the respective positioning of the lid and the multi-well plate, including the sealing rim, and a gasket. The lid is locked into place by a locking means, e.g. a "snap-lock". Sealing is accomplished by the gasket pressed against the planar surface of the sealing rim of the multi-well plate.

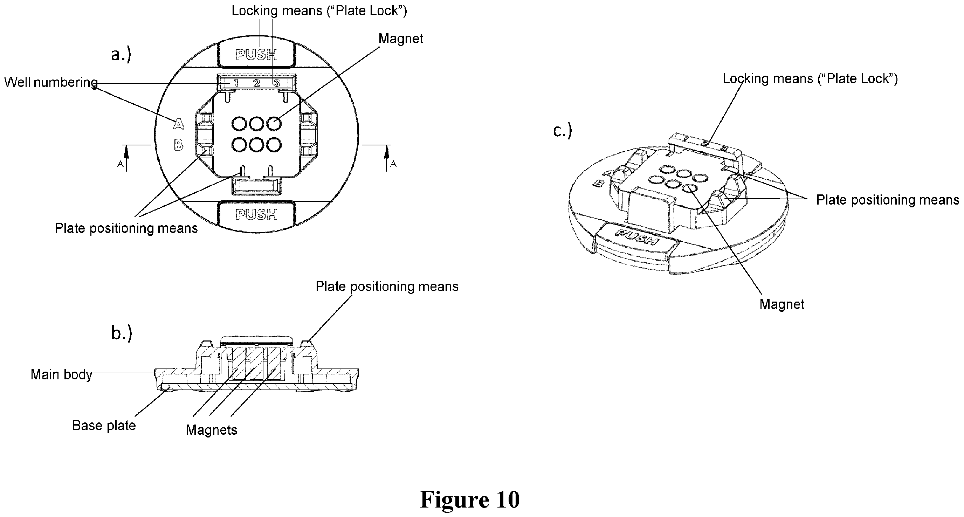

Figure 10 a) shows a top view of a of an embodiment of a device for arranging microspheres in a non-aqueous, water-immiscible liquid phase in a multi-well plate and for excluding such microspheres from contact with or exposure to a gas phase. Such a device is herein also sometimes referred to as a "magnetic rack". The line A in figure 10 a) indicates the cut surface for the cross section shown in figure 10 b) which shows a cross section of magnetic rack with the main rack body equipped with magnets and mounted on top of a base plate, such as a metal base plate. The main body features positioning means to support proper placement of a multi-well plate onto the rack. Figure 10 c) shows an isometric view of the magnetic rack.

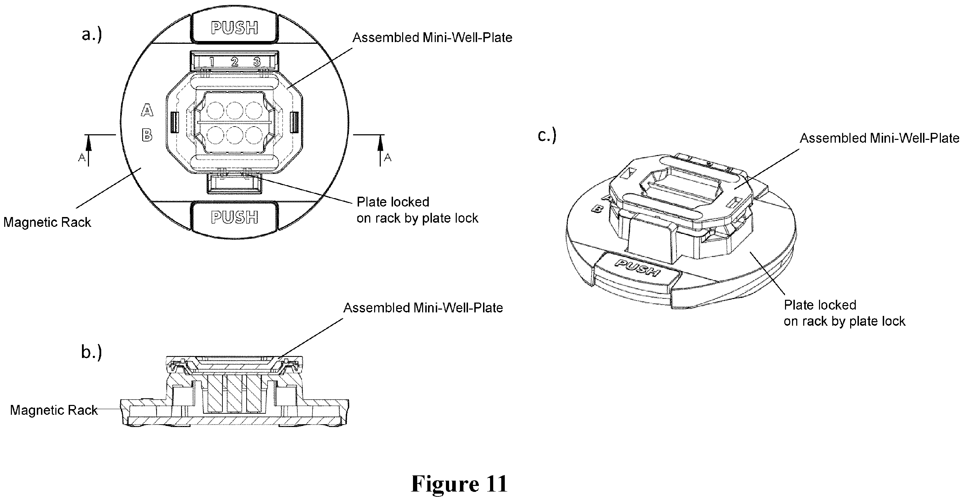

Figure 11 a) shows a top view of an embodiment of a magnetic rack with an assembled multi-well plate. Line A indicates the cut surface for the cross section shown in figure 11 b) which is the cross section of magnetic rack with a mounted multi-well plate assembly. Figure 11 c) shows an isometric view of a magnetic rack with mounted multi-well plate. The push-label on the plate indicates the operation of the unit. By pushing both labelled parts, the locking means is moved to the release-position. After properly placing the multi-well plate guided by the positioning means onto the rack, the locking means is released and snapped into position, trapping parts extruding from the main body of the multi-well plate.

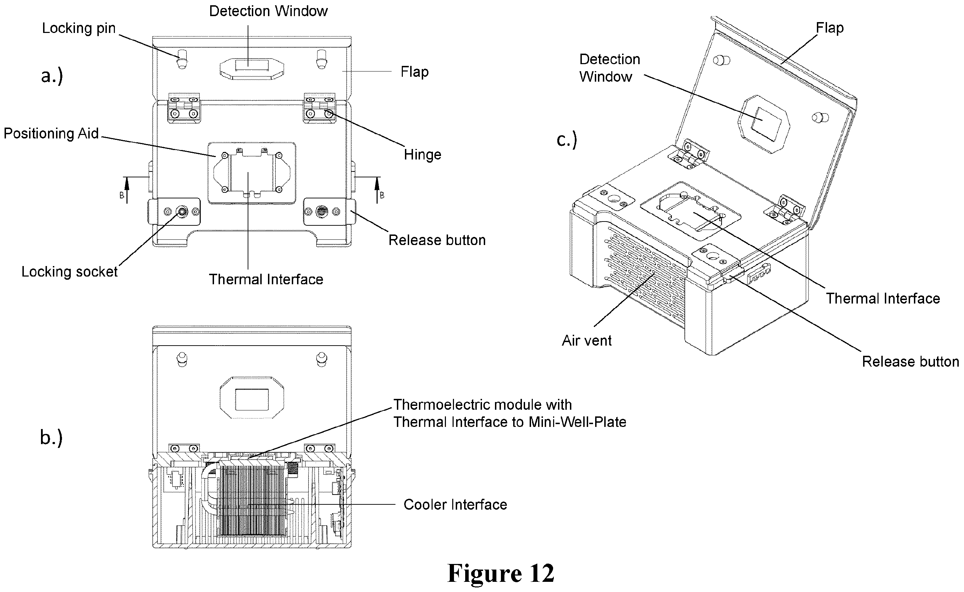

Figure 12 shows of an embodiment of a device for incubating microspheres in a multi-well plate, such device having a main body and a receptacle located in such main body, to receive such multi-well plate. Furthermore, the device comprises a flap that can be switched between an open and a closed state. When the flap is open, the device is ready to receive a multi-well plate. Line B in figure 12 a) indicates the cut surface for the cross section which is shown in figure 12 b) which shows the presence of a temperature controlling unit, e.g. a thermoelectric module with a thermal interface at the bottom of the receptacle of the device, as well as a cooler and interface. The thermoelectric module is a specific embodiment of a temperature-controlling device that may be used in accordance with the present invention. Figure 12 c) shows an isometric view of the instrument with an open flap.

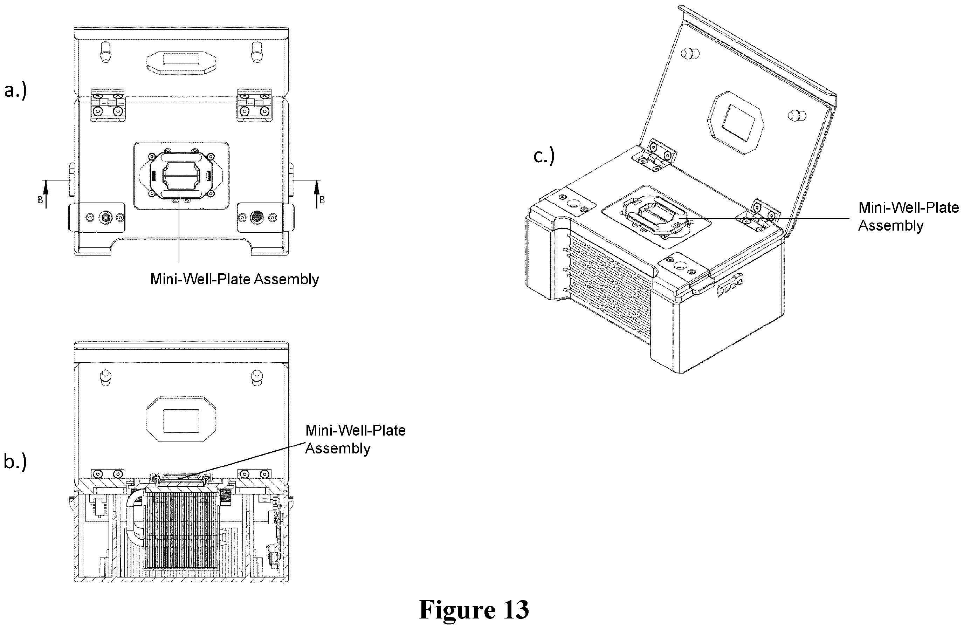

Figure 13 shows the same instrument/device as in figure 12, but this time including a multi-well plate.

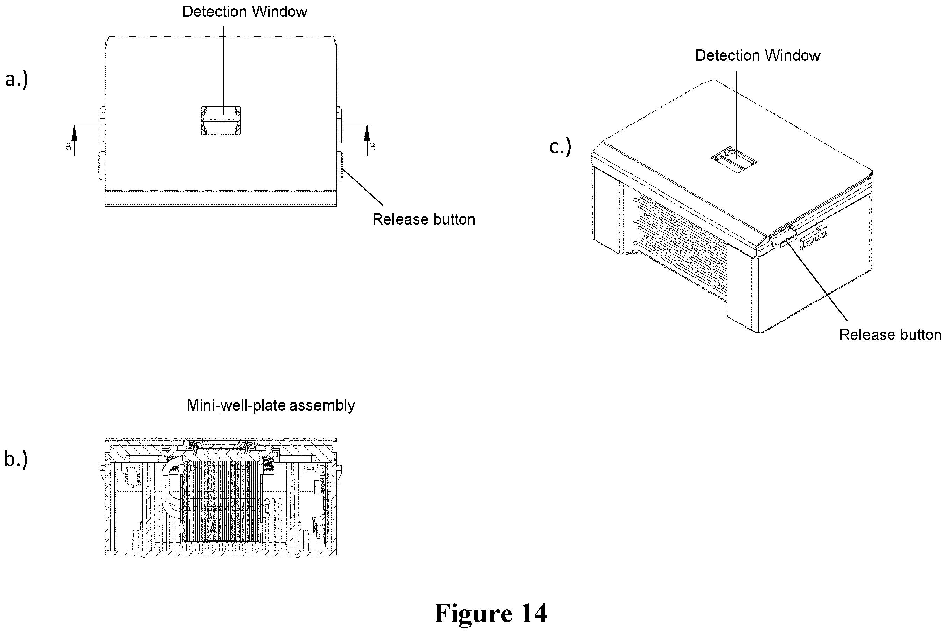

Figure 14 shows the same device as in figures 12 and 13, but with the flap this time being closed. Also shown in this figure is a release button allowing the release of the flap and opening thereof.

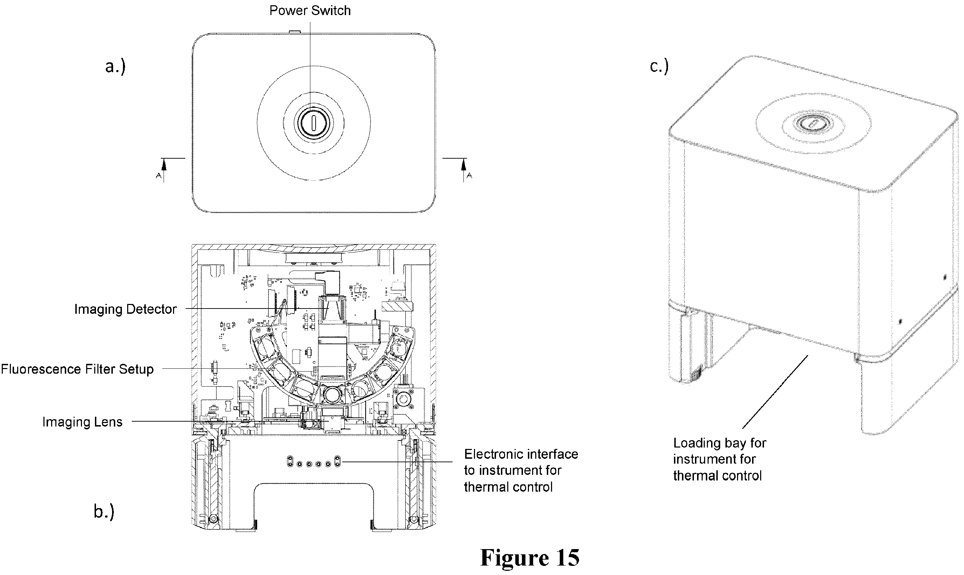

Figure 15 shows of an embodiment of a device for detecting microspheres arranged in a non-aqueous, water-immiscible liquid phase in a plurality of incubation chambers within a multi-well plate. Detection, in this embodiment occurs by fluorescence detection. The line A in figure 15a) indicates the cut surface for the cross section shown in figure 15 b). Figure 15 b) additionally shows part of the optics that is used including an imaging lens, a (fluorescence) filter setup and an imaging detector as well as an electronic interface of this device to the device/instrument for incubating said microspheres. Figure 15 c) shows an isometric view of the instrument/device and explicitly shows a loading bay for the instrument for incubating in the form of an open space/slot into which the device for incubating is inserted.

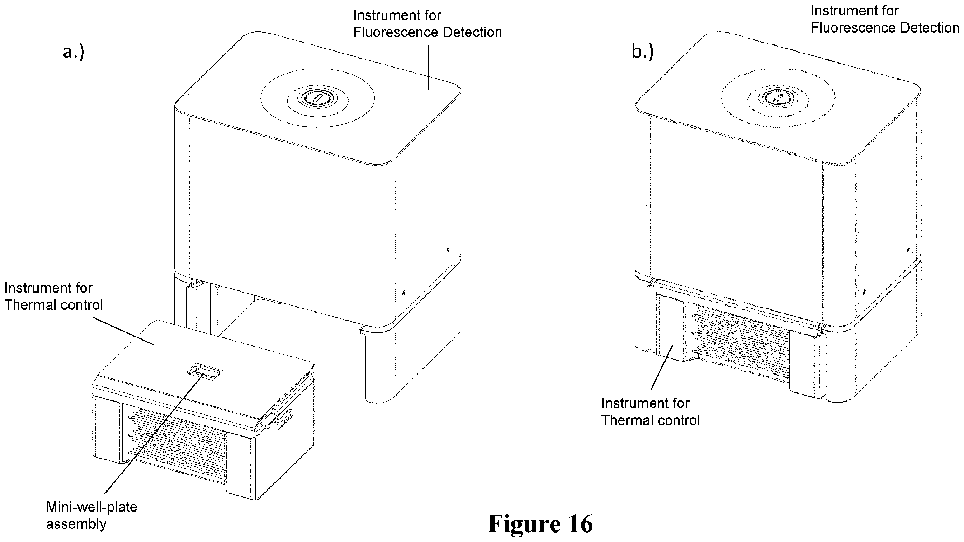

Figure 16 shows both the device for detecting as well as the device for incubating wherein in figure 16 a) the incubation device has not yet been moved into the loading bay of the detection device, whereas in figure 16 b) it has.

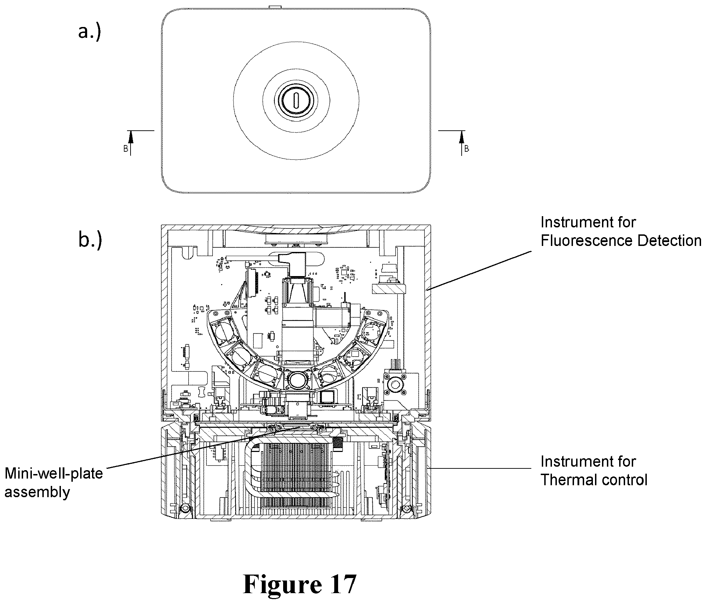

Figure 17 shows the combination of devices of figure 16 wherein the device for incubating has been inserted into the device for detecting. Figure 17 a) at line B indicates the cut surface for the cross section shown in figure 17 b). From figure 17 b) it can be seen that also the multi-well plate, and any microspheres arranged therein appropriately, are included as well.

Moreover, reference is made to the examples which are given to illustrate, not to limit the present invention.

### Examples

### Performing transcription and polymerase chain reaction within bead microspheres in a multi well plate with subsequent detection of the bead microsphere monolayer

### Bead microsphere loading

Hydrated bead microspheres (40 µl bead bed, 100 µm bead diameter, see table) both with incorporated magnetic particles were loaded with polymerase chain reaction (PCR) reagents in an aqueous environment using the same volume of a double concentrated master mix (see table) supplemented with template and an according primer-probe set by incubating 5 min at room temperature with agitation.

**Table 1: Bead and PCR mix composition**

| | Example 1 (Figure 6) | Example 2 (Figure 7) |
|---|---|---|
| Bead microsphere composition | Agarose (as described for example in WO 2018/122162A1 and WO2021/122579A1 | Agarose coupled with Cy5 dye with crosslinked chitosan (as described in European patent application no. 21206745.8) |
| Magnetic particles | 1 µm (Magnefy COOH, Bangslabs) 6× 10⁶ particles/µl beads | |
| PCR Master Mix (1×) | 100 mM Tris | |
| | 22 mM NH₄Cl | |
| | 22 mM KCl | |
| | 2.4 mM MgCl₂ | |
| | 0.1% BSA (w/v) for molecular biology BSA (Sigma) | |
| | pH8.9 | |
| | 200 µM dNTPs (biotechrabbit GmbH) | |
| | 0.2 units Hot Start Taq DNA Polymerase (biotechrabbit GmbH) | |
| RTase including RNase Inhibitor | 1 unit Reverse Transcriptase including RNase inhibitor (biotechrabbit GmbH) | - |
| Template | MS2 bacteriophage RNA, 2173 copies | human genomic DNA, 1 µg |
| Target | MS2 genome | RPP30 (copy reference gene) |
| Primer & TaqMan Probe Set | For: CTCTGAGAGCGGCTCTATTGGT (SEQ ID NO: i) | PrimePCR^{™} Probe Assay RPP30, Human, FAM Bio-Rad Laboratories, Inc. |
| | Rev: GTTCCCTACAACGAGCCTAAATTC (SEQ ID NO: 2) | |
| | Probe: Atto550-TCAGACACGCGGTCCGCTATAACGA-BHQ2 (SEQ ID NO: 3) | |
| | 400 nm each, Metabion International | |
| Segmentation Dye | 400 nM FAM | - |

### Suspension and dispersion of bead microspheres

Bead microspheres were gently sedimented using a centrifuge with swing-out rotors at 300 rpm for 1 min, the supernatant was discarded followed by addition of 100 µl separation oil (Novec 7500^{™} including 5% PicoSurf, SphereFluidics). The tube containing the prepared mix underwent heavy agitation for 15 s at a Minilys personal homogenizer (Bertin instruments, intermediate speed) to force the aqueous bead microspheres to build a stable suspension within the fluorophilic oil, a process promoted by the containing surfactant agent. As the applied oil has a density of ~1,6 g/ml, the aqueous bead microspheres float to the surface of the oil. The engulfed microspheres were washed three times with separation oil to minimise the amount of microemulsion (emulgated water and air in oil) occurred alongside the suspension process.

### Loading and assembling of the multi well cartridge

The multi well plate was placed on the loading rack which contains magnets under each well position and was flooded with 740 µl filling oil (Novec 7500^{™} with 0.5% PicoSurf). Subsequently, 3 µl engulfed bead microspheres were pipetted in each well. The magnets of the loading rack kept the superparamagnetic bead microspheres at its place within the individual wells. A tailor-made bead plate (coverslip 23 × 29 mm, 500 µm thickness) was applied on the multi well plate by placing it upright on one side of the plate and slowly tilting the coverslip over the whole plate. The vitreous bead plate submerged within the filling oil volume. Thereby, the wells were separated and covered without any air bubbles within the wells. A lid containing a gasket at the sealing rim was applied on the multi well plate in the same manner and closed by firm pressure to ensure latching of the both-sided snap locks. The lid did not come into direct contact with the bead plate while filling oil was in the interspace.

### Mounting and thermocycling

At this state the cartridge was liquid-tight and bead microspheres were captured in their respective well. Hence, the complete cartridge could be removed from the loading rack and be processed to both a thermal control unit and optical detection unit. The multi well plate was brought into a thermocycling chamber consisting of a Peltier element, electronic control and cooling unit. Mounting of the multi well plate in this chamber goes along with an applied mechanical force pressing the plate and lid together resulting in a firm fitting of the squeeze-tight gasket increasing the gas sealing. The PCR cycling was conducted after reverse transcription (if RTase present: 10 min, 50°C), preheating (2 min, 80°C) and initial denaturation (2 min, 98°C) steps as following: MS2 40× cycles 30s 98°C & 30s 57°C, RPP30 45× cycles 5s 98°C & 15s 59°C. The applied heat in the initial denaturation PCR step led to partial melting of the gasket, generating an additional long-lasting heat-sealing of the cartridge. After the final PCR step, the cartridge was actively cooled to 20°C and ready for image acquisition.

### Imaging

At each chamber position images were acquired for each channel adding up to a total of 2×25 images. One channel is required for specific amplification signals (see table) and the second channel for bead microsphere segmentation. Automated image acquisition is triggered by the BLINK toolbox software and is achieved by using an upright fluorescence microscope (Zeiss AxioZoom) equipped with a 20× objective (field of view 5.633 × 3.538 mm) and SOLA SE V-nIR light engine (Lumencor). The microscope was further equipped with three fluorescence filter sets (Cy5 ET F46-006, Cy3 ET F46-004, FITC HC F36-502, AHF Analysentechnik) and an automated x-y stage to which the thermocycler with the reaction chamber was mounted.

**Table 2: Channel overview**

| | Example 1 | Example 2 |
|---|---|---|
| Segmentation channel | FAM (λexc = 495 nm) | Cy5 (λexc = 646 nm) |
| PCR signal | Atto550 (λexc = 554 nm) | FAM (λexc = 495 nm) |

### Bead Segmentation and Endpoint Analysis

All images acquired are subjected to an automated multifaceted image processing algorithm developed by BLINK to detect and segment individual nanoreactor beads. Features including fluorescence signals in each channel, position, diameter/volume, etc. of all segmented nanoreactors are subsequently collected at individual level allowing extensive statistics. Experimental data is processed by the open-source software Jupyter.

## Claims

1. A method for arranging microspheres in a non-aqueous, water-immiscible liquid phase in an incubation chamber and for excluding such microspheres from contact with or exposure to a gas phase, said method comprising the steps:
a) Providing, in any order, a suspension comprising magnetic microspheres suspended in a non-aqueous, water-immiscible liquid phase, and an incubation chamber;
said incubation chamber having a closed bottom, an open top, at least one wall, and a defined volume; said open top being spaced apart from said closed bottom by a defined distance; said chamber being dimensioned to accommodate a defined volume of said suspension therein;
said magnetic microspheres having a first defined density; said non-aqueous, water-immiscible liquid phase having a second defined density, wherein said first defined density < said second defined density; and wherein, preferably, said suspension has a defined concentration of microspheres;
b) Filling said incubation chamber with said suspension by placing said suspension into said incubation chamber, such that in said incubation chamber, at the open top or at a first position along said defined distance between said open top and said closed bottom, there is formed a first interface between said non-aqueous, water-immiscible liquid phase and a gas phase, such gas phase surrounding said incubation chamber;
c) Removing said magnetic microspheres from said first interface or keeping them removed therefrom by attracting said magnetic microspheres to the closed bottom of said incubation chamber or to said at least one wall, wherein said attracting is achieved by exerting a magnetic force across said closed bottom or across said at least one wall of said incubation chamber;
d) Whilst keeping said magnetic microspheres attracted to the closed bottom or to said at least one wall of said incubation chamber by continuing to exert a magnetic force across said closed bottom or said at least one wall, placing a solid surface at said open top of said incubation chamber or at a position along said defined distance between said open top and said closed bottom of said incubation chamber; wherein such placing occurs such that said solid surface is in contact with said liquid phase and such that at said open top or at said position there is formed a second interface between said non-aqueous, water-immiscible liquid phase and said solid surface, wherein such second interface replaces or eliminates said first interface and thereby excludes said gas phase from being in contact with said liquid phase;
e) Allowing the magnetic microspheres to assemble at the second interface by ceasing to exert a magnetic force across said bottom or across said at least one wall of the incubation chamber, thereby releasing said microspheres from said bottom or from said at least one wall.

2. The method according to claim 1, wherein steps b) and c) are performed concomitantly or in a temporally overlapping manner.

3. The method according to any of the foregoing claims, wherein said exerting a magnetic force across said closed bottom or said at least one wall of said incubation chamber in step c) occurs by either placing a magnet in vicinity to said bottom or said at least one wall of said incubation chamber or vice versa, such that by means of said magnet, a magnetic force is exerted across the bottom or said at least one wall, wherein, preferably, in step c), said bottom or said at least one wall of said incubation chamber is in physical contact with said magnet, or in such a contact that said magnet can exert a magnetic force thereacross.

4. The method according to any of the foregoing claims, wherein by step d), said incubation chamber becomes closed in that said open top is converted into a closed top by said solid surface.

5. The method according to any of the foregoing claims, wherein in step d), said solid surface is placed at said open top of said incubation chamber or at a position along said defined distance between said open top and said closed bottom of said incubation chamber, by:
- either placing only a lid at said open top of said incubation chamber or at said position,
- or placing, firstly a separate solid surface which is not a lid, e.g. a flat plate, at said open top of said incubation chamber or at said position, and thereafter placing a lid on top of said separate solid surface.

6. The method according to any of the foregoing claims, wherein said continuing to exert said magnetic force in step d) occurs by either keeping a magnet in vicinity to said bottom or said at least one wall of said incubation chamber or vice versa, such that by means of said magnet, a magnetic force continues to be exerted across the bottom or across said at least one wall, wherein, preferably, in step d), said bottom or said at least one wall of said incubation chamber is in physical contact with said magnet, or at least in such a contact that enables said magnet to exert a magnetic force thereacross.

7. The method according to any of the foregoing claims, wherein step e) is performed by removing the magnet from the vicinity of said bottom or of said at least one wall, or vice versa, thereby stopping a magnetic force from being exerted across the bottom or across said at least one wall of said incubation chamber.

8. The method according to any of the foregoing claims, wherein assembling of said microspheres at said second interface in step e) is facilitated by actively generating convection in said incubation chamber, preferably thermal convection or mechanical convection.

9. The method according to any of the foregoing claims, wherein step e) is facilitated by exposing the bottom of said incubation chamber to a temperature controlling device, preferably by thermally contacting said bottom of said incubation chamber with said temperature controlling device, more preferably by physically contacting said bottom of said incubation chamber with said temperature controlling device.

10. The method according to any of the foregoing claims, wherein said method is a method for arranging said microspheres in a layer of microspheres along said second interface, and wherein said solid surface that is placed in step d) at said open top of said incubation chamber or at said position along said defined distance between said open top and said closed bottom of said incubation chamber, is a planar solid surface.

11. The method according to claim 10, wherein said layer is a monolayer of microspheres along said second interface.

12. The method according to any of claims 10 - 11, wherein packing of microspheres in said layer is selected from random packing, regular packing, and close packing, in particular hexagonal close packing.

13. The method according to any of claims 10 - 12, wherein packing of microspheres in said layer is close packing, in particular hexagonal close packing.

14. The method according to any of claims 10 - 13, wherein the number of magnetic microspheres filled into said incubation chamber in step b) is chosen such it does not exceed the maximum number of microspheres that can be arranged in a layer along said second interface which layer has the closest possible packing; and/or is chosen such that the magnetic microspheres, when arranged in a layer in the closest possible packing, cover an area that is equal to or smaller than the area provided by said planar solid surface which is in contact with said liquid phase.

15. The method according to any of the foregoing claims, wherein said microspheres are mono-disperse, preferably having a coefficient of variation (CV) < 15%, more preferably a CV <10%, even more preferably a CV < 5%.

16. The method according to any of the foregoing claims, wherein said solid surface, preferably said planar solid surface comprises a region which is transparent and allows for optical probing and detection of said liquid phase and any microspheres located underneath said region.

17. The method according to any of the foregoing claims, wherein said incubation chamber is a well in a multi-well plate, and wherein said multi-well plate has a plurality of such incubation chambers, as defined in any of the foregoing claims, which are arranged in said multi-well plate, preferably arranged regularly spaced apart in said multi-well plate.

18. The method according to claim 17, wherein said method is performed with two or more wells in said multi-well plate.

19. A method for incubating microspheres in a non-aqueous, water-immiscible liquid phase within an incubation chamber or within a multi-well plate, said method comprising the steps:
f) performing the method according to any of the foregoing claims, preferably according to any of claims 16 - 18;
g) performing an incubation reaction with said microspheres, wherein, optionally, said incubation reaction involves one or several changes of temperature of said microspheres.

20. A method for detecting and optically probing microspheres in a non-aqueous, water-immiscible liquid phase within an incubation chamber or within a multi-well plate, said method comprising the steps:
h) performing the method according to claim 16 or the method according to any of claims 17 - 18, when dependent on claim 16;
optionally i) performing an incubation reaction with said microspheres, wherein, optionally, said incubation reaction involves one or several changes of temperature of said microspheres;
k) detecting and optically probing said microspheres through said transparent region of said planar solid surface, preferably using a suitable detection module.

21. A multi-well plate configured to be used in a method according to any of claims 1 - 20, said multi-well plate comprising:
- a flat body having a flat surface and comprising a plurality of wells, each well having a closed bottom, an open top, at least one wall, and a defined volume; each well being dimensioned to accommodate a defined number of magnetic microspheres or a defined volume of a suspension comprising magnetic microspheres suspended in a non-aqueous, water-immiscible liquid phase therein; wherein said wells are embedded in saidflat surface of said flat body, which flat surface is configured to be contacted by a planar solid surface, such that each well becomes closed by such planar solid surface;
- a sealing rim at the circumference of said flat body, said sealing rim surrounding said flat body of said multi-well plate and protruding therefrom;
- a lid configured to become attached to and placed on top of said multi-well plate and being dimensioned to cover and seal said multi-well plate, said lid having lower surface which is a planar solid surface; said lid having a circumference and comprising a gasket at its circumference which interacts with said sealing rim of said flat body and seals said multi-well plate, when said lid is placed on top of the multi-well plate; and
- optionally a flat plate having a lower surface which is a planar solid surface, said flat plate being configured to be placed on top of said flat body and being dimensioned to cover said flat body.

22. The multi-well plate according to claim 21, wherein each well becomes closed by a planar solid surface, such planar solid surface being provided by either said lower surface of said lid or said lower surface of said flat plate, if present.

23. The multi-well plate according to any of claims 21 - 22, further comprising a flat plate having a lower surface which is a planar solid surface, said flat plate being configured to be placed on top of said flat body and being dimensioned to cover said flat body.

24. The multi-well plate according to any of claims 21 - 23, wherein said lid and, if present, said flat plate, (each) comprises (comprise) a region covering the plurality of wells, which region is optically transparent and allows for optical probing and detection of any of the wells located underneath said region(s).

25. The multi-well plate according to any of claims 21 - 24, further comprising locking means configured to lock said lid in a position on top of said multi-well plate, and optionally configured to also lock said flat plate, if present, on top of said flat body of said multi-well plate, such that said multi-well plate becomes sealed.

26. The multi-well plate according to any of claims 21 - 25, wherein said flat body is made of or comprises a plastic, preferably a plastic having elemental carbon as an additive, more preferably a plastic having elemental carbon as an additive at a proportion of > 50 wt.%, even more preferably said plastic being polycarbonate having elemental carbon as an additive at a proportion of > 50 wt. %.

27. A device for arranging microspheres in a non-aqueous, water-immiscible liquid phase in an incubation chamber and for excluding such microspheres from contact with or exposure to a gas phase, said device comprising:
- a receptacle to receive an incubation chamber or a multi-well plate; said receptacle having a bottom surface and at least one positioning means for positioning said incubation chamber or said multi-well plate on said bottom surface, said positioning means protruding from such bottom surface, said receptacle having dimensions to accommodate said incubation chamber or said multi-well plate in a snug fit therein; said incubation chamber having a closed bottom, an open top, at least one wall, and a defined volume; said multi-well plate being as defined in any of claims 21 - 26;
- a magnet or a set of magnets located at said bottom surface of said receptacle and being incorporated therein, or located at said positioning means and being incorporated therein, said magnet or magnets being configured to exert a magnetic force across said closed bottom of said incubation chamber or across the bottom of each well in said multi-well plate, or across said at least one wall of said incubation chamber across said at least one wall of each well in said multi-well plate;
- optionally, a locking means for locking said incubation chamber or said multi-well plate in said receptacle, preferably on said bottom surface in said receptacle.

28. A device for incubating microspheres in a non-aqueous, water-immiscible liquid phase within an incubation chamber or within a multi-well plate, said device comprising:
- a main body having an upper surface and a receptacle configured to receive a multi-well plate in accordance with any of claims 21 - 26, said receptacle being located in said upper surface, said receptacle having a bottom;
- a temperature controlling device located at and/or incorporated in said bottom of said receptacle and being configured to thermally and/or physically contact the bottom of an incubation chamber or the bottom of each well of a multi-well plate, when inserted into said receptacle; and
- a lid or flap, attached to the main body and being configured to cover the upper surface of said main body and to be placed on top of said receptacle and said incubation chamber or said multi-well plate, when placed into said receptacle of said device; wherein said lid or flap is further configured to lock said incubation chamber or said multi-well plate in said receptacle when said lid or flap is placed on top of said receptacle, wherein said lid or flap furthermore comprises an optically transparent region which comes to be located on top of said receptacle and thereby allows optical probing of an incubation chamber or multi-well plate that is present in said receptacle.

29. The device according to claim 28, further comprising, in said receptacle, an incubation chamber or a multi-well plate according to any of claims 21 - 26.

30. A device for detecting and optically probing microspheres in a non-aqueous, water-immiscible liquid phase within an incubation chamber or within a multi-well plate, said incubation chamber or said multi-well plate being located in a device for incubating according to claim 28, said device for detecting comprising:
- a housing comprising a detection module, said detection module comprising an image detector, one or several filters and at least one imaging lens;
a loading bay configured to receive and accommodate a device for incubating according to any of claims 28 - 29, said loading bay comprising an interface for electronically connecting to a device for incubating according to any of claims 28 - 29, wherein said loading bay is located within the housing such that the detection module can be used to detect and optically probe said device for incubating when present in said loading bay, preferably can be used to detect and optically probe an incubation chamber or multi-well plate, located in said device for incubating microspheres; wherein detection and optical probing occurs through said optically transparent region of said lid or flap of said device for incubating; and wherein said optically transparent region of said lid or flap of said device for incubating is aligned with
• said optically transparent region(s) of said lid and said flat plate of said multi-well plate, if such incubation chamber or multi-well plate is present in said device for incubating,
• said image detector,
• one filter of said one or several filters, and
• said at least one imaging lens;
such that optical probing occurs through said transparent region of said lid or flap of said device for incubating and through said optically transparent region(s) of said lid and said flat plate of said multi-well plate if present in said device for detecting.
